# EUROPÄISCHE PATENTSCHRIFT

(11) **EP 0 876 140 B1**
(45) Veröffentlichungstag und Bekanntmachung des Hinweises auf die Patenterteilung: **15.10.2003**
(21) Anmeldenummer: 97914098.5
(22) Anmeldetag: 27.01.1997
(51) Int. Cl.: A61K 9/08, A61K 49/00, A61P 43/00, A61P 37/00

(54) **VERBESSERTE KONTRASTMITTENLÖSUNGEN FÜR DIE INTRAVASALE ANWENDUNG**
IMPROVED KONTRAST AGENT SOLUTIONS FOR INTRAVENOUS ADMINISTRATION
SOLUTIONS D'AGENTS DE CONTRASTE AMELIOREES POUR ADMINISTRATION INTRAVEINEUSE

(30) Priorität: 25.01.1996 DE 19604095
(43) Veröffentlichungstag der Anmeldung: 11.11.1998
(73) Patentinhaber: SCHERING AKTIENGESELLSCHAFT, 13342 Berlin (DE)
(72) Erfinder: SPECK, Ulrich, D-13465 Berlin (DE); SCHUHMANN-GIAMPIERI, Gabriele, D-12203 Berlin (DE); MUSCHICK, Peter, D-16321 Ladeburg (DE); KRAUSE, Werner, D-13505 Berlin (DE)
(86) Internationale Anmeldenummer: DE9700170
(87) Internationale Veröffentlichungsnummer: WO97026862

(56) Entgegenhaltungen:
- EP-A- 0 260 811
- EP-A- 0 437 622
- WO-A-89/09614
- DE-A- 4 446 694
- FR-A- 2 435 253

## Beschreibung

### Erfindungsgegenstand

Die Erfindung betrifft die in den Patentansprüchen gekennzeichneten Gegenstände, das heißt die Verwendung bestimmter Substanzen als Zusätze zu Kontrastmittellösungen, zur Herstellung von Arzneioder Diagnosemitteln, zur vermeidung oder Verminderung verzögerter Überempfindlichkeitsreaktionen.

### Hintergrund der Erfindung

Für einige therapeutische und diagnostische medizinische Anwendungen werden größere Volumina konzentrierter und/oder viskoser Lösungen intravasal appliziert. Beispiele sind vor allem Infusionen zur Verabreichung großer Mengen von Nährstoffen, osmotisch oder kolloidosmotisch wirksamer Substanzen und Kontrastmittel. Typischerweise werden von diesen Lösungen 0.5-20 ml/kg Körpergewicht appliziert; die Lösungen enthalten in der Summe 10 oder mehr Gewichtsprozent Wirk- und Hilfsstoffe und sind häufig viskoser als das Blut. Zum Teil handelt es sich auch um niedriger konzentrierte, niedriger dosierte Emulsionen, Suspensionen, oder Zubereitungen komplexer Strukturen wie Micellen oder Liposomen. Die Lösungen verursachten früher eine Vielzahl unterschiedlicher Nebenwirkungen, von denen die Akutreaktionen im Vordergrund stan en. Solche Wirkungen betrafen direkte metabolische oder toxische Effekte der verabreichten Substanzen, Wirkungen bedingt durch zum Teil extrem hohe und unphysiologische Osmolalität der Lösungen, unphysiologische und schädliche Lösungsmittel, Stabilisatoren und Puffer. Außerdem traten akute allergische oder allergieartige Ereignisse auf. Inzwischen sind die meisten dieser unerwünschten Wirkungen in ihrer Ursache erkannt und durch bessere Wirkstoffe und Zubereitungen vermieden worden. Dadurch wurde die Häufigkeit und der Schweregrad von Nebenwirkungen ganz wesentlich reduziert. Untersuchungen der letzten Jahre haben jedoch gezeigt, daß ein früher eher seltenerer, jedenfalls weniger auffälliger Typ von Nebenwirkungen zunehmend Beachtung findet und Probleme bereitet. Es handelt sich dabei um allergieartige oder sonstige wenig dosisabhängige, oft unerklärliche sofort oder verzögert (d.h. Stunden bis Tage) nach der Verabreichung auftretende Überempfindlichkeitsreaktionen, die sich in Hautrötungen, Urticaria, Quaddeln, Ödamen, Schieirnhautschweflungen und anderen Symptomen äußern und sich - allerdings selten - bis hin zu schweren Atembeschwerden und Schockzuständen steigern können.
Gegenstand der Erfindung sind daher Zusätze zu hochkonzentrierten und/oder viskosen Lösungen sowie Mikropartikel enthaltenden Zubereitungen zur intravasalen Applikation, die akute oder verzögerte Überempfindlichkeitsreaktionen vermeiden oder in Häufigkeit und Intensität vermindern. Weiterhin wird die Verwendung von Zusätzen zu wirkstoffhaltigen Lösungen zum Zwecke der Verminderung von akuten oder verzögerten Überempfindlichkeitsreaktionen beschrieben.

### Stand der Technik

Zusätze zu Injektions- und Infusionslösungen sind Stand der Technik. Verwendet werden Elektrolyte, Zucker und Zuckeralkohole wie Mannit zur Anpassung der Osmolalität hypotoner WirkstoBlösungen an die Osmolalität des Blutes. Ausnahmsweise wurden Elektrolyte den Wirkstofflösungen auch dann zugesetzt, wenn die Osmolalität dadurch über diejenige des Blutes hinaus erhöht wird (WO 90/11094). Zweck dieser Zusätze ist eine Verbesserung der lokalen Verträglichkeit bei Kontakt der betreffenden Wirkstofflösungen mit Blutgefäßen und Geweben, insbesondere am Herzen. Weiterhin sind Stabilisatoren und Puffer gebräuchlich. Röntgenkontrastmitteln wurde zum Teil vor Gebrauch Heparin oder andere gerinnungshemmende Substanzen zugesetzt, um einen erhöhten Gerinnungsschutz beim Zurückströmen von Blut in vorher kontrastmittdgefüllte Katheter und Spritzer zu gewährleisten [Jackson, D.M.A. und P. Dawson: Current usage of contrast agents, anticoagulant and antiplatelet drugs in angiography and angioplasty in the U.K., Clinical Radiology 50, 699-704, (1995); Miller, D.L.: Heparin in angiography: Current pattems of use, Radiology 172, 1007-1011, (1989), WO 94/14478]. Diese Mischungen finden ebenfalls vor allem bei der Verabreichung in Arterien oder zur röntgenologischen Darstellung von Venen Verwendung, wo es auf eine lokale und nicht-systemische Schutzwirkung der Gerinnungshemmer im Hinblick auf die Bildung von Thromben ankommt. Der Zusatz von Lokalanästhetika zu Röntgenkontrastmitteln zur Schmerzlinderung ist durch die Entwicklung weniger stark hypertoner Präparate überholt. Vasodilatatoren, Prostacycline, Harnstoff und andere Substanzen wurden zur Verbesserung der Mikrozirkulation oder zur Wirkungsverstärkung von Kontrastmitteln eingesetzt oder empfohlen (DE 4 135 193, DE 4 446 694). Schließlich wurden gelegentlich Kontrastmittellösungen therapeutisch wirksamen Substanzen oder Präparaten zugesetzt, um deren Verteilung im Gewebe oder deren Abstömen im Blutgefäßsystem sichtbar zu machen. Infusionslösungen wurden zum Verdünnen von Therapeutika eingesetzt. Als Röntgenkontrastmittelzusätze gebräuchlich sind bisher schon Na₂Ca EDTA (als Stabilisator, allerdings nur bis ca. 2 mmolar), Na Citrat und Tris(hydroxymethyl)-methylamin ("Tris")/HCl (als Puffer, im Bereich von 10 bis 20 mmolar) und anorganische und organische Basen einschließlich basischer Aminosäuren (als Gegenion für Kontrastmittelsäuren).Die Bemühungen in den vergangenen 30 Jahren richteten sich eher auf eine Verminderung dieser Zusätze, um eventuelle unerwünschte physiologische Effekte zu venneiden. Physiologische anorganische Ionen wurden zur Anpassung des Elektrolytmilieus zugesetzt und Papaverin und Prostacycline zur Verbesserung der Gefiißdrrstellung, Lokalanästhetica zur Schmerzlinderung und Harnstoff zur Verbesserung der Darstellung der Nieren und Harnwege durch Röntgenkontrastmittel. Eine Übersicht wurde u.a. von S.H. Kim, H.K. Lee und M.C. Han: "Incompatibility of water-soluble contrast media and intravascular pharmacologic agents", an invitro study, Investigative Radiology 27 45-49, (1992) publiziert Eine geringe Assoziierung der Röntgenkontrastmittelmoleküle in der zu verabreichenden Lösung wurde als nachteilig gewertet, da sie zu einer unphysiologischen Erhöhung der Osmolalität führt.

### Aufgabenstellung

Die hier zu lösende Aufgabe hat erst in den letzten Jahren an Bedeutung gewonnen. Die ersten Publikationen über systematische Untersuchungen zu verzögerten Überempfindlichkeitsreaktlonen nach Kontrastmittelgabe stammen aus der Mitte der 80er Jahre. Die Häufigkeit dieser Reaktionen hat offenbar mit der Verbesserung der akuten Verträglichkeit der modernen Präparate eher zugenommen. Auffällig ist, daß die neueren nichtionischen Röntgenkontrastmittel gerade auch weniger allergieartige akute Nebenwirkungen auslösen als die früher gebräuchlichen stark hypertonen ionischen Kontrastmittel. Umso überraschender und unverständlicher ist der Anstieg der Häufigkeit von verzögerten Überempfindlichkeitsreaktionen, die zu einer wachsenden Zahl von Publikationen und zu erheblicher Beunruhigung der Anwender geführt hat. Unter verzögerten Reaktionen werden solche Nebenwirkungen verstanden, die erst eine oder mehrere Stunden nach Verabreichung des betreffenden Mittels auftreten. Es gibt bisher keine schlüssige Hypothese über Ursache und Mechanismus des Auftretens der verzögerten Reaktionen. Es ist darüber hinaus auch nicht klar, ob einige der akuten Nebenwirkungen durch einen ähnlichen Mechanismus bewirkt werden, wie die verzögerten Reaktionen.
Aufgabe der Erfindung ist es daher Zusätze zu Kontrastmitteln zur Verfügung zu stellen die akute oder verzögerte Überempfindlichkeitsreaktionen wesentlich vermindern oder sogar vollständig vermeiden.

### Beschreibung der Erfindung

Die Verminderung und Vermeidung verzögerter Überempfindlichkeitsreaktionen wird durch Zusatz bestimmter physikalisch oder pharmakologisch wirkender Substanzen zu den ansonsten weitgehend unveränderten Kontrastmittellösungen (im Folgenden auch als Infusions- oder Injektionslösungen bezeichnet) erreicht. Die eigentlich wirksamen Bestandteile der Lösungen, d.h. die kontrastgebenden Substanzen oder Strukturen, werden nicht verändert.

Als Zusätze geeignet erwiesen sich zwei Gruppen von Substanzen:
a) Substanzen, die die Selbstassoziation der Wirkstoffmoleküle oder Wirkstoffkomponenten in den hochkonzentrierten Infusionslösungen vermindern.
   In diese Klasse von Substanzen gehören Aminosäuren, wie Glycin, Leucin, Lysin, Asparagin und Asparaginsäure, Phenylalanin, Tryptophan und deren neutrale, physiologisch verträgliche Salze und Amide. Die Konzentration der Zusätze zum Zwecke der Entassoziation der Wirkstoffinoleküle ist so zu wählen, daß ein deutlicher Effekt in dieser Hinsicht auftritt, ohne daß allerdings die Osmolalität der Lösungen physiologisch sehr problematische Werte erreicht (zum Beispiel 3000 mosm/kg H₂O) und ohne daß toxikologisch bedenkliche Konzentrationen oder Dosierungen der Zusätze erreicht werden oder die Viskosität der Lösung allein durch die hohe Konzentration der Zusätze wieder wesentlich ansteigt. Ein solcher entassoziierender Effekt ist beispielsweise an einer meßbaren Viskositätsminderung der Lösung trotz Zusatzes eines oder mehrerer Stoffe (d.h. trotz Erhöhung der Gesamtkonzentration der Lösung) zu erkennen.
   Besonders geeignete Konzentrationen der Zusätze bewegen sich im Bereich von 1 mmolar bis ca. 2000 mmolar, bevorzugt ist der Bereich von 5 mmolar bis 1000 mmolar. Unberücksichtigt bleiben die zur Pufferung und Stabilisierung von Röntgenkontrastmitteln gebräuchlichen Substanzen im Konzentrationsbereich bis ca. 30 mmolar. Die entassoziierende Wirkung der Zusätze läßt sich entweder über eine Messung der Viskosität der Lösungen, die Messung der Osmolalität oder andere bekannte physikalische Meßverfahren erfassen.
b) Substanzen mit einer Wirksamkeit auf immunologische Vorgänge.
   Es erwiesen sich solche Substanzen als geeignet, die das Komplementsystem hemmen. Verschiedene in diesem Sinne wirksame Substanzen und Substanzklassen wurden zum Beispiel von Asghar, S.S.: Pharmacological Reviews 36 223-244, (1984) beschrieben. Die Verwendung dieser Substanzen soll jedoch nicht im Sinne einer systemischen oder lokalen Prophylaxe oder Therapie erfoigen. Erreicht wird ein lokaler Effekt während der Mischungs- und Verdünnungsvorgänge bei und nach der Infusion oder Injektion konzentrierter Wirkstofflösungen. Nur während dieser Zeit und nur während des Verdünnungsvorganges liegen lokal ausreichende Konzentrationen der Zusätze vor, die eine Initiierung von Überempfindlichkeitsreaktionen verhindern. Die mit den Wirkstofflösungen verabreichten Dosierungen an Hemmstoffen des Komplementsystems sind im allgemeinen nicht ausreichend, um längere Zeit anhaltende und/oder generalisierte protektive oder therapeutische Effekte zu erzielen. Im Gegenteil, der direkte Zusatz der Substanzen zu den Infüsions- oder Injektionslösungen zur Verhinderung von Überempfindlichkeitsreaktionen erlaubt es, so niedrige Dosierungen zu wählen und die Verabreichung zeitlich so weit zu beschränken, daß es nicht zu einer ansonsten bei den Patienten oft unerwünschten Beeinträchtigung der Immunabwehr oder der physiologischen Crerinnungsvorgänge kommt. Beispiele geeigneter Zusätze sind ε-Aminocapronsäure, Lysin, Arginin, Ornithin, Cystein, Homocystein, Peptide (zum Beispiel Tryptophan-Tyrosin, Glutathion), Polylysin, Polyinosinsäure, Suranun, Chlorpromazin und Mesoporphyrin. Davon sind einige Vertreter bereits in der Vergangenheit als Zusätze oder Bestandteile von Röntgenkontrastmitteln verwendet worden. So ist es bekannt, daß man den Röntgenkontrastmitteln für die Darstellung von Blutgefäßen mittels Katheter Heparin zusetzen kann, um die Gerinnung von Blut zu verhindern, das ggf. in die Katheter und Spritzen zurückfließt. Die Anwendung ist nur sinnvoll, wenn längere Pausen zwischen Einzelinjektiönen eintreten. Bei intravenöser Verabreichung wurde ein gerinnungihenimender Zusatz bisher nicht diskutiert, da ein Zurückströmen von Blut in Überleitungsschläuche und Spritzen anderweitig verhindert wird, Die Notwendigkeit dieses Zusatzes ist selbst in der Arteriographie umstritten. Auch Aminosäuren sind als Bestandteile von Röntgenkontrastmitteln eingesetzt oder empfohlen worden. Dabei handelt es sich um die basischen Aminosäuren, die als Gegenionen für jodhaltige Röntgenkontrastmittelsäuren Verwendung gefunden haben. Sie wurden vereinzelt anstelle der gebräuchlicheren Kationen wie Na⁺ oder Meglumin⁺ eingesetzt. Für das Lysinsalz der Amidotrizoesäure wurde auch eine bessere Verträglichkeit gegenüber den anderen Salzen beschrieben. Diese bezog sich aber nicht auf verzögerte Überempfindlichkeitsreaktionen.

Die Konzentration der betreffenden Zusätze. in den Infusions- Und Injektionslösungen kann so niedrig gewählt werden, daß systemische Effekte ausbleiben, gering oder jedenfalls nicht für die protektive Wirkung entscheidend sind. Beispiele sind folgende:

| | |
|---|---|
| ε-Aminocapronsäure | 0.01 - 20 mg/ml |
| Lysin-HCl | 0.1 - 100 mg/ml |
| Polylysin | 0.01 - 10 mg/ml |
| Mesoporphyrin | 0.01 - 2mg/ml |
| Glutathion | 0.1 - 50 mg/ml |

Von besonderem Interesse ist die Möglichkeit, unterschiedliche Effekte gleichzeitig mit ein und demselben Zusatzstoff zu erreichen. Mit ε-Aminocapronsäure ebenso wie mit den übrigen Aminosäuren werden mehrere der zuvor genannten erwünschten Wirkungen gleichzeitig erzielt. Dadurch wird vermieden, daß die Infüsions- oder Injektionslösungen in ihrer Zusammensetzung zu komplex werden. Ebenso sind diejenigen Zusätze bevorzugt, die nicht nur verzögerten Überempfindlichkeits-reaktionen vorbeugen sondern gleichzeitig andere nützliche Funktionen in den Infusions- oder Injektionslösungen ausüben; indiesem Sinne genannt seien Viskositätsverminderung, Stabilisierung der Lösungen, Pufferung und bestimmte erwünschte zusätzliche phannakologische Effekte. Ein wesentlicher Vorteil der erfindungsgemäßen Zusätze liegt darin, daß in den meisten Fällen keine zusätzliche Behandlung der Patienten erforderlich ist. Die mit der Infusions- oder Injektionslösung verabreichten Zusatzstoffe wirken sofort und gleichzeitig mit ihrer Injektion und zwar auch bei einmaliger Gabe. Die protektive Wirkung wird bei deutlich niedrigerer Gesamtdosis der Zusätze erzielt als diese für eine getrennte Prophylaxe oder Therapie von Überempfindlichkeitsreaktionen notwendig wäre. Viele der in Kombination mit den Infüsions- oder Injektionslösungen wirksamen Zusätze sind separät verabreicht pharmakologisch weitgehend inert; sie üben jedenfalls allein keine bemerkenswerte Wirkung auf das Immunsystem aus sondern vermindern nur die unerwünschten Wirkungen konzentrierter Infüsions- und Injektionslösungen. Die oben beschriebenen Zusätze bewirken eine Verminderung der Aktivierung des Immunsystems auf unterschiedlichen Stufen und eine Verminderung unerwünschter pharmakologischer Effekte. Es kommt zu einer Verringerung der Aufnahme des eigentlichen Wirkstoffs der Infusions- oder Injektionslösungen in immunologisch relevante Zellen und Gewebe. Schließlich werden die eingangs beschriebenen akuten und verzögerten Überempfindlichkeitsreaktionen in Häufigkeit und Ausmaß vermindert und die Vetträglichkeit insgesamt verbessert.

### Beispiele

Die folgenden Ausführungsbeispiele von Formulierungen sollen den Erfindungsgegenstand erläutern, ohne ihn darauf zu beschränken.

### Beispiel 1

59,803 g Iotrolan und 0,010 g Na₂Ca EDTA und 0,040 g NaHCO₃ und 2,250 g Glycin werden in Aqua dest gelöst, so daß ein Endvolumen von 100 ml bei 20 °C erreicht wird. Die fertige Lösung wird in einer fest verschlossenen Infusionsflasche fiir 20 Minuten bei 121 °C autoklaviert. Die Osmolalität der Lösung bei 37 °C beträgt 724 mosm/kg H₂O die Dichte 1,327 g/ml und die Viskosität 6,96 mPas jeweils bei 37 °C.

### Beispiele 2

59,803 g Iotrolan und 0,010 g Na₂Ca EDTA und 2,193 g Lysin werden in Aqua dest gelöst, so daß ein Endvolumen von 95 ml bei 20 °C erreicht wird. Der pH-Wert wird mit 1nHCl auf 7.2 eingestellt. Es wird auf 100 ml Lösung aufgefüllt. Die fertige Lösung wird in einer fest verschlossenen Infusionsflasche für 20 Minuten bei 121 °C autoklaviert. Die Lösung hat eine Osmolalität von 543 mosm/kg H₂O, eine Dichte von 1,315 g/ml und eine Viskosität von 6,2 mPas jeweils bei 37 °C.

### Beispiele 3

59,803 g Iotrolan und 0,010 g Na₂Ca EDTA und 1,46 g Lysin werden in Aqua dest gelöst, so daß ein Endvolumen von 95 ml bei 20 °C erreicht wird. Der pH-Wert wird mit InHCl auf 7.2 eingestellt. Es wird auf 100 ml Lösung aufgefüllt. Die fertige Lösung wird in einer fest verschlossenen Infusionsflasche für 20 Minuten bei 121 °C autoklaviert. Die Lösung hat eine Osmolalität von 445 mosm/kg H₂O, eine Dichte von 1,311 g/ml und eine Viskosität von 6,0 mPas jeweils bei 37 °C.

### Beispiele 4

59,803 g lotrolan und 0,010 g Na₂Ca EDTA und 3,07 g Glutathion werden in Aqua dest gelöst, so daß ein Endvolumen von 95 ml bei 20 °C erreicht wird. Der pH-Wert wird mit 1 nHCl bzw. 1 n NaOH auf 7.2 eingestellt. Es wird auf 100 ml Lösung aufgefüllt. Die fertige Lösung wird in einer fest verschlossenen Infusionsflasche für 20 Minuten bei 121 °C autoklaviert. Die Lösung hat eine Osmolalität von 455 mosm/kg H₂O, eine Dichte von 1,312 g/ml und eine Viskosität von 6,2 mPas jeweils bei 37 °C.

### Beispiele 5

78,886 g Iopromid und 0,010 g Na₂Ca EDTA und 0,292 g Lysin werden in Aqua dest gelöst, so daß ein Endvolumen von 95 ml bei 20 °C erreicht wird. Der pH-Wert wird mit 1nHCl auf 6.8 eingestellt. Es wird auf 100 ml Lösung aufgefüllt. Die fertige Lösung wird in einer fest verschlossenen Infusionsflasche für 20 Minuten bei 121 °C autoklaviert. Die Lösung hat eine Osmolalität von 780 mosm/kg H₂O, eine Dichte von 1,403 g/ml und eine Viskosität von 9,7 mPas jeweils bei 37 °C.

### Beispiel 6

64,7 g Iohexol und 0,010 g Na₂Ca EDTA und 0,121 g Tris und 5,7 mg Mesoporphyrin werden in Aqua dest gelöst, so daß ein Endvolumen von 95 ml bei 20 °C erreicht wird. Der pH-Wert wird mit 1nHCl auf 7.4 eingestellt Es wird auf 100 ml Lösung aufgefüllt. Die fertige Lösung steril filtriert. Die Lösung hat eine Osmolalität von 670 mosm/kg H₂O, eine Dichte von 1,344 g/ml und eine Viskosität von 5,9 mPas jeweils bei 37 °C.

## Patentansprüche

1. Verwendung von Aminosäuren, von deren Salzen und Amiden als Zusätze zu Kontrastmittellösungen in einer Menge, die über die für eine Substitution von im Kontrastmittel-Molekül vorhandenen aciden Wasserstoffatomen hinausgeht zur Herstellung von Arznei- oder Diagnosemitteln, zur Vermeidung oder Verminderung verzögerter Überempfindlichkeitsreaktionen.

2. Verwendung von Hemmstoffen der Komplementaktivierung, ausgenommen Heparin, in einer Konzentration von 0,01 - 100 mg/ml als Zusätze zu konzentrierten oder partikeihaltigen Kontrastmittellösungen zur Herstellung von Arznei- oder Diagnosemitteln, zur Vermeidung oder Verminderung akuter oder verzögerter Überempfindlichkeitsreaktionen.

3. Verwendung nach Anspruch 1, wobei die Konzentration der Aminosäuren, deren Salze und Amide eine Konzentration von 1 bis 2000 mMol/l beträgt.

4. Verwendung nach Anspruch. 1 zu Lösungen nichtionischer Kontrastmittel.

5. Verwendung nach den Ansprüchen 1 und 2 in Lösungen dimerer nichtionischer Kontrastmittel.

6. Verwendung gemäß Anspruch 1 von Glycin, Leucin, Lysin, Asparagin, Asparaginsäure, Phenylalanin oder Tryptophan und deren Salze als Aminosäuren.

7. Verwendung nach Anspruch 1 von ε-Aminocapronsäure, Lysin, Arginin, Ornithin, Cystein, Homocystein, Peptiden, Polypeptiden, Tryptophan-Tyrosin, Glutathion, Polylysin, Polyinosinsäure, Suramin, Chlorpromazin oder Mesoporphyrin als Hemmstoffe der Komplementaktivierung bzw. als gerinnungshemmende Stoffe Substanzen.

## Claims

1. Use of amino acids, of their salts and amides as additions to contrast media solutions in an amount exceeding that for replacement of acidic hydrogen atoms present in a contrast medium molecule for producing medicaments or diagnostic aids for preventing or diminishing delayed hypersensitivity reactions.

2. Use of inhibitors of complement activation, excepting heparin, in a concentration of 0.01 - 100 mg/ml as additions to concentrated or particle-containing contrast media solutions for preventing or diminishing acute or delayed hypersensitivity reactions.

3. Use according to Claim 1, where the concentration of amino acids, their salts and amides amounts to a concentration of from 1 to 2000 mmol/1.

4. Use according to Claim 1 to solutions of nonionic contrast media.

5. Use according to Claims 1 and 2 in solutions of dimeric nonionic contrast media.

6. Use according to Claim 1 of glycine, leucine, lysine, asparagine, aspartic acid, phenylalanine or tryptophan and their salts as amino acids.

7. Use according to Claim 1 of ε-aminocaproic acid, lysine, arginine, ornithine, cysteine, homocysteine, peptides, polypeptides, tryptophantyrosine. glutathione, polylysine, polyinosinic acid, suramine, chlorpromazine or mesoporphyrin as inhibitors of complement activation or as anticoagulant substances.

## Revendications

1. Utilisation d'acides aminés, de leurs sels et amides comme additifs de solutions d'agent de contraste en une quantité qui dépasse la quantité pour une substitution des atomes d'hydrogène acides présents dans la molécule d'agent de contraste pour la préparation de médicaments ou d'agents diagnostiques en vue d'éviter ou de diminuer des réactions d'hypersensibilité retardée.

2. Utilisation de substances inhibitrices de l'activation de compléments, à l'exception de l'héparine, en une concentration de 0,01 à 100 mg/ml comme additifs de solutions d'agent de contraste concentrées ou contenant des particules pour la préparation de médicaments ou d'agents diagnostiques en vue d'éviter ou de diminuer des réactions d'hypersensibilité aiguë ou retardée.

3. Utilisation selon la revendication 1, la concentration en acides aminés, leurs sels et amides présentant une concentration de 1 à 2000 mM/l.

4. Utilisation selon la revendication 1 dans des solutions d'agents de contraste non ioniques.

5. Utilisation selon les revendications 1 et 2 dans des solutions d'agents de contraste dimères non ioniques.

6. Utilisation selon la revendication 1 de glycine, leucine, lysine, asparagine, acide aspartique, phénylalanine ou tryptophane et leurs sels comme acides aminés.

7. Utilisation selon la revendication 1 d'acide ε-aminocaprique, de lysine, d'arginine, d'ornithine, de cystéine, d'homocystéine, de peptides, de polypeptides, de tryptophane-tyrosine, de glutathion, de polylysine, de poly(acide inosinique), de sumarine, de chloropromazine ou de mésoporphyrine comme substances inhibitrices de l'activation de compléments ou, selon le cas, comme substances inhibitrices de la coagulation.
